# EUROPÄISCHE PATENTSCHRIFT

(11) **EP 1 094 056 B1**
(45) Veröffentlichungstag und Bekanntmachung des Hinweises auf die Patenterteilung: **25.02.2004**
(21) Anmeldenummer: 00120644.0
(22) Anmeldetag: 21.09.2000
(51) Int. Cl.: C07C 67/42, C07C 69/017, C07C 45/46, C07C 49/825

(54) **Verfahren zur Herstellung von monoacetylierten Hydrochinonverbindungen**
Process for the preparation of monoacetylated hydrochinone compounds
Procédé de préparation de composés de l'hydroquinone monoacétylés

(30) Priorität: 20.10.1999 DE 19950616
(43) Veröffentlichungstag der Anmeldung: 25.04.2001
(73) Patentinhaber: BASF AKTIENGESELLSCHAFT, 67056 Ludwigshafen (DE)
(72) Erfinder: Schein, Karin, Dr., 67065 Ludwigshafen (DE); Baldenius, Kai-Uwe, Dr., 67227 Frankenthal (DE); Siegel, Wolfgang, Dr., 67117 Limburgerhof (DE); Stürmer, Rainer, Dr., 67127 Rödersheim-Gronau (DE); Ruff, Detlef, Dr., 67067 Ludwigshafen (DE); Jaedicke, Hagen, Dr., 67069 Ludwigshafen (DE)

(56) Entgegenhaltungen:
- FR-A- 2 655 335
- US-A- 3 377 241
- H.H.LEE ET AL.: "Synthesis of Flavones from Lindera lucida" JOURNAL OF THE CHEMICAL SOCIETY., April 1965 (1965-04), Seiten 2743-2749, XP002158562 CHEMICAL SOCIETY. LETCHWORTH., GB
- Beisltein abstract of compound with Reg. nr. 2650725
- Beilstein abstract of compound with Reg. nr. 2116336
- Beilstein abstract of compound with Reg. nr. 28849085
- Beisltein abstract of compound with Reg. nr. 4315232l

## Beschreibung

Die vorliegende Erfindung betrifft ein Verfahren zur Herstellung von monoacetylierten Hydrochinonverbindungen sowie die neuen Endprodukte des Verfahrens.

Hydrochinonverbindungen sind gesuchte Zwischenprodukte zur Herstellung von Naturstoffen, Vitaminen und Carotinoiden. Beispielsweise dient 2,3,6-Trimethylhydrochinon zur industriellen Totalsynthese von α-Tocopherol (Vitamin E) (Ullmann's Enzcyclopedia of Industrial Chemistry, 5^{th} edition, Vol. A27, S. 484 f., 4.11.2).

Für die Herstellung von Vitamin-E-Verbindungen mit olefinischen, isoprenoiden Seitenketten wie Tocotrienol ist ein anderer Syntheseweg nötig, da die bei der Herstellung von Tocopherol. üblichen sauren Synthesebedingungen zur Isomerisierung oder Cyclisierung der olefinischen Seitenkette führen (P. Karrer, H. Reutschler, *Helv. Chim. Acta* 1944, *27*, 1297; H.J. Kabbe, A. Widdig, *Angew. Chem. Int. Ed. Engl.* 1982, *21*, 247-256, P. Schudel, H. Mayer, J. Metzger, R. Rüegg, O. *Isler, Helv. Chim. Acta* 1963, *46*, 2517).

Die Synthese von Tocotrienolen aber auch Tocopherolen kann beispielsweise durch Umsetzung von Dihydroxyacetophenonverbindungen und E,E-Farnesylaceton unter basischen Bedingungen zu den entsprechenden 4-Oxo-Tocotrienolen (H. J. Kabbe et al., *Angew. Chem. Int. Ed. Engl.* 1982, *21,* 247-256; B.C. Pearce et al., *J. Med. Chem.* 1994, *37,* 526) und anschließender Reduktion der 4-Oxo-Tocotrienole zu den Tocotrienolen (H. J. Kabbe, H. Heitzer, *Synthesis* 1978, 888; B. C. Pearce et al., *J. Med. Chem.* 1994, *37*, 526-541) erfolgen.

Monoacetylierte Hydrochinonverbindungen, die durch Verseifung mit beispielsweise methanolischen Natriumhydroxid in die entsprechenden Dihydroxyacetophenonverbindungen überführbar sind (N. Cohen et al, J. Org. Chem 1978, 43 (19), 3723-3726), stellen deshalb besonders gesuchte Zwischenprodukte dar.

FR 2 655 335 offenbart ein Verfahren zur Herstellung von einfach substituierten Hydrochinonmonoacetaten durch Acetylierung von einfach substituierten Phenolen und anschließender Umsetzung mit Peroxocarbonsäuren.

Es ist bekannt, am Position 4-Sauerstoff monoacetyliertes 2-Acetyl-3,5,6-Trimethylhydrochinon aus 2,3,6-Trimethylhydrochinon durch Umsetzung mit BF_{3/}Essigsäure herzustellen (N. Cohen et al, J. Org. Chem 1978, 43 (19),3723-3726).

Dieses Verfahren hat den Nachteil, daß als Edukte bereits Hydrochinone eingesetzt werden, die über ein aufwendiges Verfahren mit mindestens zwei Syntheseschritten aus wohlfeilen Edukten wie Phenolen hergestellt werden müssen (Ullmann's Enzcyclopedia of Industrial Chemistry, 5^{th} edition, Vol. A27, S. 485, 4.11.3)

Es stellte sich daher die Aufgabe, den geschilderten Mängeln abzuhelfen und ein neues Verfahren zur Herstellung von monoacetylierten Hydrochinonverbindungen mit vorteilhaften Eigenschaften bereitzustellen.

Demgemäß wurde ein Verfahren zur Herstellung von monoacetylierten Hydrochinonverbindungen der Formel I 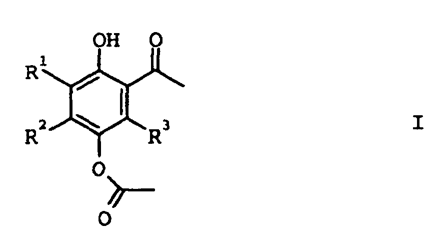 gefunden, wobei
R¹, R² oder R³ unabhängig voneinander Wasserstoff oder Methyl bedeuten, indem man Diacetylphenolverbindungen der Formel II 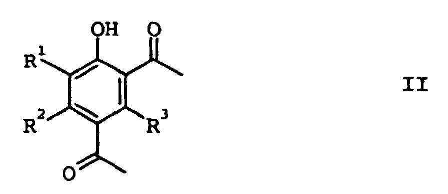 mit Peroxoverbindungen, umsetzt.

Es war nicht zu erwarten, daß sich die Oxidation von asymmetrisch acetylierten Phenolen mit hoher Selektivität bezogen auf eine Acetylgruppe in hohen Ausbeuten durchführen läßt.

Höfle et al. beschreiben die Monooxidation von einem symmetrisch diacyliertem Dimethoxyphenol mittels Bayer-Villiger Oxidation (Liebigs Ann. Chem. 1984, 1883-1904).

Ferner ist von einer anderen Substanzklasse bekannt, daß die Umsetzung von 3,5-Diacetyl-1,2,4-Trimethoxybenzol mit Peressigsäure mit 30% Ausbeute 5-Acetoxy-3-Acetyl-1,2,4-Trimethoxybenzol als Nebenprodukt liefert (H.H. Lee et al., J. Chem. Soc. 1965, 2743-2749).

Erfindungsgemäß werden unter Peroxoverbindungen, anorganische oder organische Verbindungen verstanden, die eine Peroxidgruppe enthalten. Bevorzugte Peroxoverbindungen sind beispielsweise H₂O₂ oder Persäuren oder deren Salze.

H₂O₂ kann im erfindungsgemäßen Verfahren beispielsweise als wäßrige Lösung eingesetzt werden.

Bevorzugte Persäuren sind beispielsweise anorganische Persäuren oder gegebenfalls substituierte Carbonpersäuren, wie beispielsweise gegebenenfalls substituierte Arylpersäuren oder gegebenenfalls substituierte C₁-C₄-Alkylpersäuren.

Als Substituenten der vorstehend erwähnten Carbonpersäuren kommen beispielsweise NO₂ oder Halogen in Betracht.

Bevorzugt sind gegebenenfalls halogenierte Carbonpersäuren, wie beispielsweise gegebenenfalls halogenierte Arylpersäuren oder gegebenenfalls halogenierte C₁-C₄-Alkylpersäuren.

Unter halogenierten Carbonpersäuren werden Carbonpersäuren verstanden, die mit bis zu 6 gleichen oder verschiedenen Halogenresten, wie beispielsweise F, Cl, Br oder I, vorzugsweise F oder Cl substituiert sein können.

In einer bevorzugten Ausführungsform des Verfahrens können die gegebenenfalls substituierten Carbonpersäuren in situ unter Verwendung von H₂O₂ und der entsprechenden, gegebenenfalls substituierten Carbonsäure hergestellt werden.

Bevorzugte, gegebenenfalls halogenierten C₁-C₄-Alkylpersäuren sind beispielsweise Perameisensäure, Peressigsäure, Trifluorperessigsäure oder Monopermaleinsäure.

Bevorzugte, gegebenenfalls substituierte Arylpersäuren sind beispielsweise Perbenzoesäure, m-Chlorperbenzoesäure, 3,5-Dinitroperbenzoesäure, p-Nitrobenzoesäure, Monoperphthalsäure.

Als bevorzugte Salze der gegebenenfalls substituierten Carbonpersäuren kommen deren Alkali- oder Erdalkalimetallsalze, wie beispielsweise das Magnesiumsalz der Monoperphthalsäure in Frage.

Bevorzugte anorganische Persäuren bzw. deren Salze sind beispielsweise Peroxosulfonsäuren, wie H₂S₂O₈ oder H₂SO₅ bzw. deren Alkali- oder Erdalkalimetallsalze, wie beispielssweise K₂S₂O₈ oder Peroxophophorsäuren, wie H₄P₂O₈ oder H₃PO₅ oder Natriumperborate, wie beispielsweise NaBO₃.

Als besonders bevorzugte Peroxoverbindungen eignen sich H₂O_{2.} oder m-Chlorperbenzoesäure im erfindungsgemäßen Verfahren.

Die Diacetylphenolverbindungen der Formel II werden im erfindungsgemäßen Verfahren mit Peroxoverbindungen in Anwesenheit oder Abwesenheit einer Säure umgesetzt.

Prinzipiell kann das erfindungsgemäße Verfahren zur Herstellung von monoacetylierten Hydrochinonverbindungen der Formel I mit Peroxoverbindungen in Abwesenheit einer Säure durchgeführt werden.

Speziell bei der Verwendung von Persäuren wie beispielsweise Perameisensäure, Peressigsäure, Trifluorperessigsäure, Monopermaleinsäure, Perbenzoesäure, m-Chlorperbenzoesäure, 3,5-Dinitroperbenzoesäure, p-Nitrobenzoesäure, Monoperphtalsäure, H₂S₂O₈, H₂SO₅, H₄P₂O₈ oder H₃PO₅. als Peroxoverbindungen kann das erfindungsgemäße Verfahren zur Herstellung von monoacetylierten Hydrochinonverbindungen der Formel I in einer bevorzugten Ausführungsform vorteilhaft in Abwesenheit einer Säure durchgeführt werden.

Vorzugsweise läßt sich das Verfahren besonders vorteilhaft durchführen, wenn die Diacetylphenolverbindungen der Formel II mit Peroxoverbindungen in Gegenwart einer Säure umgesetzt werden.

Unter Säure wird erfindungsgemäß eine Brönsted- oder Lewis-Säure, ein Gemisch aus Brönsted-Säuren, ein Gemisch aus Lewis-Säuren oder ein Gemisch aus Brönsted- und Lewis-Säuren verstanden.

Bevorzugte Brönsted-Säuren sind anorganische Säuren wie beispielsweise H₂SO₄ oder HCl oder Carbonsäuren, insbesondere gegebenenfalls halogenierte Carbonsäuren, wie gegebenenfalls halogenierte C₁-C₄-Alkylcarbonsäuren, wie beispielsweise Ameisensäure, Essigsäure, Propionsäure oder Trifluoressigsäure.

Bevorzugte Lewis-Säuren sind die Halogenide der dritten Hauptgruppe, wie beispielsweise BF₃ oder AlCl₃.

Bevorzugte Säuren sind Ameisensäure und Trifluoressigsäure.

In einer besonders bevorzugten Ausführungsform des Verfahrens werden die folgenden Kombinationen aus Peroxoverbindungen und Säuren verwendet:
H₂O₂ und Ameisensäure,
H₂O₂ und H₂SO₄.
H₂O₂ und BF₃,
m-Chlorperbenzoesäure und Trifluoressigsäure,
K₂S₂O₈ und H₂SO₄,
NaBO₃ und Trifluoressigsäure oder
NaBO₃ und Trifluoressigsäure/Essigsäure.

Vorteilhafterweise kann das erfindungsgemäße Verfahren unter Zusatz eines Puffersystems, wie beispielsweise eines Na₂HPO₄-Puffers durchgeführt werden.

Das Verfahren zur Herstellung der Verbindungen der Formel I kann ohne zusätzliches Lösungsmittel oder in inerten organischen Lösungsmittel, wie beispielsweise CH₂Cl₂ oder CHCl₃ durchgeführt werden.

Für den Fall, daß das Verfahren zur Herstellung der Verbindungen der Formel I ohne zusätzliches Lösungsmittel durchgeführt wird, kann die Säure als Lösungsmittel dienen. In diesem Falls wird die Säure in einem Überschuß eingesetzt. Die Menge der zugesetzten Säure ist nicht kritisch und beträgt je nach Verdünnung typischerweise 30 bis 60 mol-äqu. bezogen auf die umzusetzende Verbindung der Formel II.

Für den Fall, daß das Verfahren zur Herstellung der Verbindungen der Formel I in Abwesenheit von Säure durchgeführt wird, dient das inerte organische Lösungsmittel als Lösungsmittel.

Desweiteren kann es vorteilhaft sein, daß Verfahren zur Herstellung der Verbindungen der Formel I in einem inerten organischen Lösungsmittel und in Anwesenheit einer Säure durchzuführen. In diesem Fall ist Menge der zugesetzten Säure ebenfalls nicht kritisch und beträgt typischerweise 0,001 mol-äqu. bis 4 mol-äqu., insbesondere 0,01 mol-äqu. bis 2 mol-äqu. bezogen auf die umzusetzende Verbindung der Formel II.

Die stöchiometrischen Mengen der eingesetzten Peroxoverbindungen sind nicht kritisch und betragen typischerweise 1 bis 10 mol-äqu., insbesondere 2 bis 4 mol-äqu. bezogen auf die umzusetzende Verbindung der Formel II.

Die Verfahrenstemperatur ist nicht kritisch und beträgt typischerweise -80°C bis 100°C, insbesondere 0°C bis 30°C.

Die Isolierung des Verfahrensprodukts der Formel I kann nach an sich bekannten Methoden erfolgen, beispielsweise durch Extraktion, Chromatographie oder destillative Methoden.

Dabei kann es vorteilhaft sein, vor der Isolierung nach Umsatzstillstand Peroxofänger wie beispielsweise Na₂S₂O₃ unter Eiskühlung zuzugeben, um die überschüssigen Peroxoverbindungen zu zerstören.

Vorzugsweise werden im erfindungsgemäßen Verfahren monoacetylierte Hydrochinonverbindungen der Formel I hergestellt, wobei
R¹ = R² = R³ = Methyl oder
R¹ = R³ = Methyl, R² = Wasserstoff oder
R¹ = R² = Methyl, R³ = Wasserstoff oder
R¹ = Methyl, R² = R³ = Wasserstoff
bedeuten.

Die Herstellung von Diacetylphenolverbindungen der Formel II 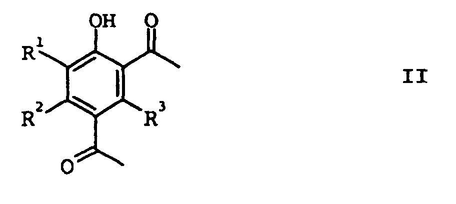 wobei
R¹, R² oder R³
unabhängig voneinander Wasserstoff oder Methyl
bedeuten, erfolgt vorzugsweise indem man Phenolverbindungen der Formel III 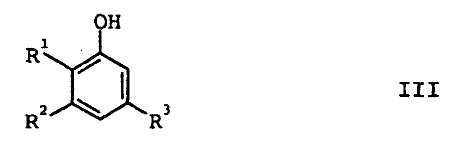 mit einem Acetylierungsmittel in Gegenwart eines sauren Katalysators umsetzt.

Es ist bekannt, Diacetylphenolverbindungen der Formel II für R¹ und R² = Methyl und R³ = Wasserstoff durch ein zweistufiges Verfahren aus der entsprechenden Phenolverbindung der Formel III herzustellen (H.-J. Knölker et al., Helv. Chim. Acta 1993, 76, 2500-2514). Dazu wird die freie Hydroxy-Gruppe von 2,3-Dimethylphenol im ersten Schritt mit Essigsäureanhydrid und Basenzugabe acetyliert. In einem zweiten Schritt wird die entstandene O-Acetyl-Verbindung durch Friesumlagerung in Ausbeuten kleiner 10 % zu 2,4-Diacetyl-5,6-dimethylphenol umgesetzt.

Das Verfahren hat den Nachteil, daß es über zwei getrennte Verfahrensschritte abläuft und das gewünschte Produkt nur als Nebenprodukt in Ausbeuten kleiner 10 % liefert.

Es war überraschend, daß die Durchführung des Verfahrens mit einem Acetylierungsmittel in Gegenwart eines sauren Katalysators, also in einem Schritt, das gewünschte Produkt in höheren Ausbeuten liefert.

Unter einem Acetylierungsmittel werden Verbindungen verstanden, die in der Lage sind eine Acetylpruppen zu übertragen.

Bevorzugte Acetylierungsmittel sind beispielsweise Essigsäure, Essigsäurehalogenide, insbesondere Essigsäurechlorid, Essigsäureanhydrid oder weitere Aktivester der Essigsäure, insbesondere Essigsäuresäure-N-hydroxysuccinimide, oder Phenolester und Halophenolester der Essigsäure.

Ein besonders bevorzugtes Acetylierungsmittel ist Essigsäurechlorid.

Unter einem sauren Katalysator werden Brönstedsäuren, vorzugsweise HF, H₂SO₄, H₃PO₄ oder HClO₄ oder Lewissäuren, vorzugsweise AlCl₃, BF₃, FeCl₃, TiCl₄, ZnCl₂, SbF₅ oder SbCl₅ verstanden.

In einer bevorzugten Ausführungsform des Verfahrens zur Herstellung von Verbindungen der Formel II verwendet man als Acetylierungsmittel ein Acetylhalogenid, insbesondere Acetylchlorid und eine Lewissäure, insbesondere AlCl₃ als sauren Katalysator.

Die stöchiometrischen Mengen der eingesetzten Reagenzien sind nicht kritisch und betragen typischerweise 1 bis 20 mol-äqu. insbesondere 4 bis 10 mol-äqu. des sauren Katalysators und 1 mol-äqu. bis 20 mol-äqu., insbesondere 2 mol-äqu. bis 8 mol-äqu. des Acetylierungsmittels, jeweils bezogen auf die umzusetzende Verbindung der Formel III.

Als Lösungsmittel eignen sich für das Verfahren zur Herstellung von Diacetylphenolverbindungen der Formel II gängige organische Lösungsmittel, insbesondere gegebenenfalls substituierte Kohlenwasserstoffe, wie beispielsweise Dichlormethan, 1,2-Dichlorethan, Nitrobenzol, Trichlorethylen oder CHCl₃.

Die Temperatur während des Verfahrens zur Herstellung von Diacetylphenolverbindungen der Formel II ist nicht kritisch und beträgt typischerweise -20°C bis 100°C. Je nach Edukt und gewählter Temperatur beträgt die Reaktionsdauer 1 bis 20 h bis zum Umsatzstillstand.

In einer bevorzugten Ausführungsform erfolgt das Verfahren zur Herstellung von Verbindungen der Formel II unter inverser Reaktionsführung. Dabei wird das Acetylierungsmittel und die Lewissäure in einem Lösungsmittel vorgelegt und die Verbindung der Formel III zugetropft. Vorteilhafterweise wird das Zutropfen so geregelt, das 20°C Reaktionstemperatur nicht überschritten werden.

Das Verfahrensprodukt der Formel II kann durch an sich bekannte Methoden wie Extraktion, Chromatographie oder destillative Methoden isoliert werden. Dabei kann es vorteilhaft sein, das Reaktionsgemisch vor der Isolierung durch Zugabe von Wasser zu quenchen.

Die vorliegende Erfindung betrifft ferner ein Gesamtverfahren zur Herstellung von monoacetylierten Hydrochinonverbindungen der Formel I, 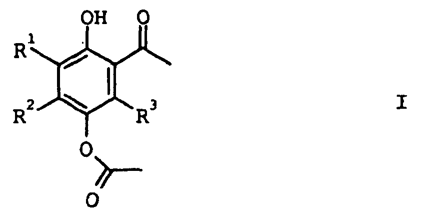 indem man Phenolverbindungen der Formel III 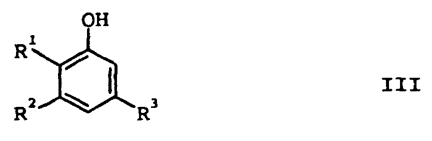 mit einem Acetylierungsmittel in Gegenwart eines sauren Katalysators zu den Diacetylphenolverbindungen der Formel II 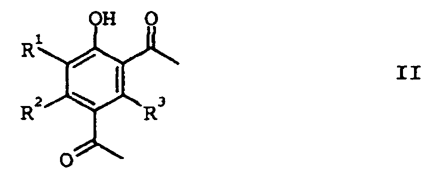 umsetzt und diese mit Peroxoverbindungen, gegebenenfalls in Gegenwart einer Säure umsetzt.

Das Gesamtverfahren kann in einem Schritt oder in zwei Schritten unter Isolierung der Zwischenprodukte der Formel II durchgeführt werden. Vorzugsweise wird das Verfahren in zwei Schritten durchgeführt.

Ferner betrifft die Erfindung Verbindungen der Formel Ia bis Ie,

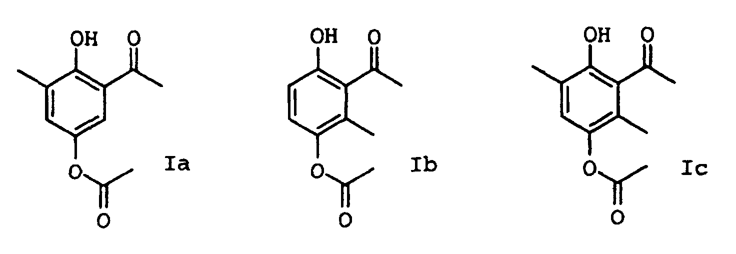

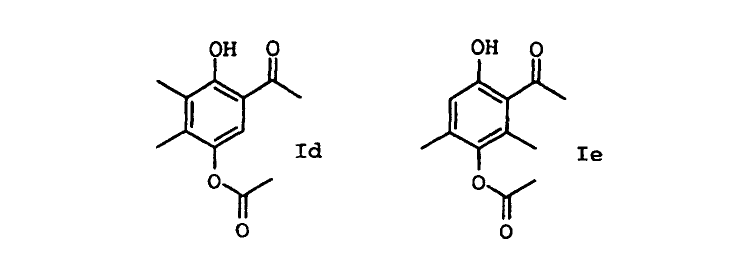

Die nachstehenden Beispiele erläutern die Erfindung:

### Beispiel 1

### Herstellung von Verbindungen der Formel II unter Verwendung von Acetylchlorid und AlCl₃

0,6 mol Aluminiumtrichlorid wurden in 240 ml 1,2-Dichlorethan vorgelegt und bei Temperaturen kleiner gleich 20°C mit 0,5 mol Acetylchlorid versetzt. Anschließend wurden 0,1 mol der Verbindung der Formel III (gelöst in 50 ml 1,2-Dichlorethan) unter Eiskühlung zugetropft, so daß 20°C nicht überschritten wurden. Dann wurde die Mischung bis zum Rückfluß erhitzt. Nach 5-13h wurde die Reaktion abgekühlt und unter Eiskühlung,mit Wasser gequencht. Die Phasen wurden getrennt, die wäßrige Phase 3 mal mit je 200 ml Methyl-t-Butylether extrahiert und die vereinigten org. Phasen mit ges. NaHCO₃-Lsg. gewaschen, bis die wäßrige Phase alkalisch blieb. Nach Waschen mit ges. NaCl-Lsg. und Trocknen über MgSO₄ wurde das Lösungsmittel am Rotationsverdampfer entfernt und der Rückstand säulenchromatographiert (Kieselgel 60 (230 bis 400 mesh); Eluent: Heptan/Essigester).

Das vorstehende Experiment wurde mit 2,3-Dimethylphenol (Beispiel 1.1) und 2-Methylphenol (Beispiel 1.2) als Verbindung der Formel III durchgeführt.

### Beispiel 1.1

### Herstellung von 2,4-Diacetyl-5,6-dimethylphenol (IId)

Die Umsetzung von 2,3-Dimethylphenol nach der in Beispiel 1 beschriebenen allgemeinen Arbeitsvorschrift lieferte 2,4-Diacetyl-5,6-dimethylphenol (IId) in 66 % Ausbeute (nach Säulenchromatographie) bei einem Umsatz von 100 %;
GC-MS: 206
¹H-NMR(CDCl₃, 400MHz) in [ppm]: 2,19 (s, 3H); 2,43 (s, 3H); 2,64 (s, 3H); 7,91 (s, 1H); 13,0 (s, 1H);
¹³C-NMR(CDCl₃, 100MHz) in [ppm]: 11,16; 18,01; 26,35; 29,67; 115,90; 127,45; 129,64; 130,40; 145,92; 162,54; 200,45; 203,98;

### Beispiel 1.2

### Herstellung von 2,4-Diacetyl-6-methylphenol (IIa)

Die Umsetzung von 2-Methylphenol nach der in Beispiel 1 beschriebenen allgemeinen Arbeitsvorschrift lieferte 2,4-Diacetyl-6-Methylphenol (IIa) in 27 % Ausbeute (nach Säulenchromatographie) bei einem Umsatz von 100 %;
GC-MS: 192
¹H-NMR:(CDCl₃, 400MHz) in [ppm]: 2,28 (s, 3H); 2,58 (s, 3H); 2,71(s, 3H); 7,91(s, 1H); 8,23 (s, 1H); 13,0 (s, 1H);
¹³C-NMR:(CDCl₃, 100MHz) in [ppm]: 15,54; 26,24; 26,76; 118,33; 127,82; 127,86; 129,61; 136,61; 164,67; 196,03; 205,02;

### Beispiel 2

### Herstellung von Verbindungen der Formel I unter Verwendung von Ameisensäure und H₂O₂

5 mmol der Verbindung der Formel II und 0,20 mol Ameisensäure wurden innerhalb von 5 Minuten mit 10 mmol H₂O₂ (30 Vol.-% in H₂O) versetzt. Anschließend wurde 8 h bei Raumtemperatur gerührt. Anschließend wurde unter Eiskühlung Na₂S₂O₃ zugegeben, mit 50 ml H₂O verdünnt und 3 mal mit je 25 ml Toluol extrahiert. Die vereinigten org. Phasen werden mit Triethylamin auf pH = 6 eingestellt, mit Wasser gewaschen, über MgSO₄ getrocknet und das Lösungsmittel am Rotationsverdampfer entfernt. Die Ausbeute wurde über GC-Flächenprozent des Rohproduktes bestimmt.

Das vorstehende Experiment würde mit 2,4-Diacetyl-5,6-dimethylphenol (IId) (Beispiel 2.1) und 2,4-Diacetyl-6-methylphenol (IIa) (Beispiel 2.2) als Verbindung der Formel II durchgeführt.

### Beispiel 2.1

### Herstellung von 2-Acetyl-4-acetoxy-5,6-dimethylphenol (Id)

Die Umsetzung von 2,4-Diacetyl-5,6-dimethylphenol (IId) nach der in Beispiel 2 beschriebenen allgemeinen Arbeitsvorschrift lieferte 2-Acetyl-4-acetoxy-5,6-dimethylphenol (Id) in 96 % Ausbeute bei einem Umsatz von 97 %;
¹H-NMR(CDCl₃, 400MHz) in [ppm]: 2,12(s,3H); 2,19 (s, 3H); 2,32(s,3H); 2,58(s,3H); 7,228 (s,1H); 12,5 (s,1H).
¹³C-NMR(CDCl₃, 100MHz) in [ppm]: 11,35; 13,71; 20,72; 26,52; 116,68; 120,05; 127,22; 158,43; 169,82; 203,69.

### Beispiel 2.2

### Herstellung von 2-Acetyl-4-acetoxy-6-methylphenol (Ia)

Die Umsetzung von 2,4-Diacetyl-6-methylphenol (IIa) nach der in Beispiel 1 beschriebenen allgemeinen Arbeitsvorschrift lieferte 2-Acetyl-4-acetoxy-6-methylphenol (Ia) in 87 % Ausbeute bei einem Umsatz von 98 %;
GC-MS: 208
¹H-NMR(CDCl₃, 400MHz) in [ppm]: 2,26(s,3H); 2,30(s,3H); 2,60(s,3H); 7,09(s,1H); 7,30(s,1H); 12,40(s,1H);
¹³C-NMR(CDCl₃, 100MHz) in [ppm]:15,58; 20,97; 26,75; 118,45; 199,99; 129,02; 130,74; 141,35; 158,72; 169,87; 204,08;

### Beispiel 3

### Herstellung von Verbindungen der Formel I unter Verwendung von Trifluoressigsäure und m-Chlorperbenzoesäure in Dichlormethan

5 mmol der Verbindung der Formel II wurden in 60 ml Dichlormethan gelöst und unter Eiskühlung zunächst mit 15 mmol m-Chlorperbenzoesäure und anschließend tropfenweise mit 5 mmol Trifluoressigsäure versetzt. Nach Zugabe wurde das Eisbad entfernt und 8 h bei Raumtemperatur gerührt. Nach Beendigung der Reaktion wurde das überschüssige Reagens mit Na₂S₂O₃ zerstört und die Reaktionsmischung mit je 25 ml ges. wäßriger NaHSO₃-Lsg., ges. wäßriger NaHCO₃-Lsg. und Wasser gewaschen und über MgSO₄ getrocknet. Nach Entfernen des Lösungsmittels wurde die Rohausbeute bestimmt.

Das vorstehende Experiment wurde mit 2,4-Diacetyl-5,6-dimethylphenol (IId) (Beispiel 3.1) als Verbindung der Formel II durchgeführt.

### Beispiel 3.1

### Herstellung von 2-Acetyl-4-acetoxy-5,6-dimethylphenol (Id)

Die Umsetzung von 2,4-Diacetyl-5,6-dimethylphenol (IId) nach der in Beispiel beschriebenen allgemeinen Arbeitsvorschrift lieferte 2-Acetyl-4-acetoxy-5,6-dimethylphenol (Id) in 85 % Roh-Ausbeute;
¹H-NMR(CDCl₃, 400MHz) in [ppm]: 2,12(s,3H); 2,19(s,3H); 2,32(s,3H); 2,58(s,3H); 7,228 (s,1H); 12,5 (s,1H).
¹³C-NMR(CDCl₃, 100MHz) in [ppm]: 11,35; 13,71; 20,72; 26,52; 116,68; 120,05; 127,22; 158,43; 169,82; 203,69.

## Patentansprüche

1. Verfahren zur Herstellung von monoacetylierten Hydrochinonverbindungen der Formel I 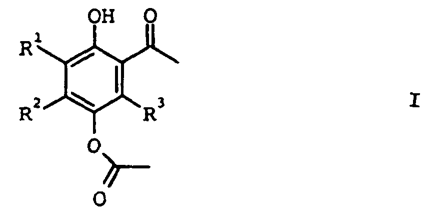 wobei
R¹, R² oder R³
unabhängig voneinander Wasserstoff oder Methyl
bedeuten, **dadurch gekennzeichnet, daß** man Diacetylphenolverbindungen der Formel II 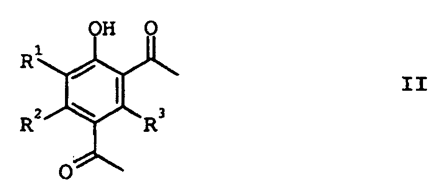 mit Peroxoverbindungen umsetzt.

2. Verfahren nach Anspruch 1, **dadurch gekennzeichnet, daß** man die Diacetylphenolverbindungen der Formel II mit Peroxoverbindungen in Gegenwart einer Säure umsetzt.

3. Verfahren nach Anspruch 2, **dadurch gekennzeichnet, daß** man als Peroxoverbindungen H₂O₂ oder Persäuren oder deren Salze verwendet.

4. Verfahren nach Anspruch 2 oder 3, **dadurch gekennzeichnet, daß** man als Säure eine, gegebenenfalls halogenierte, Carbonsäuren verwendet.

5. Verfahren nach Anspruch 1, **dadurch gekennzeichnet, daß** man die Diacetylphenolverbindungen der Formel II herstellt, in dem man Phenolverbindungen der Formel III 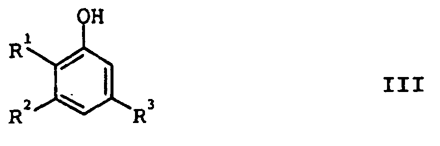 mit einem Acetylierungsmittel in Gegenwart eines sauren Katalysators zu den Diacetylphenolverbindungen der Formel II 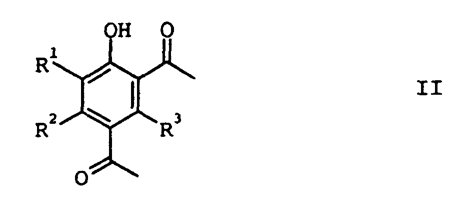 umsetzt.

6. Verbindungen der Formel Ia bis Ie,

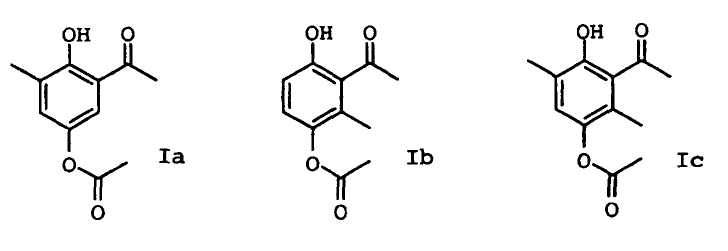

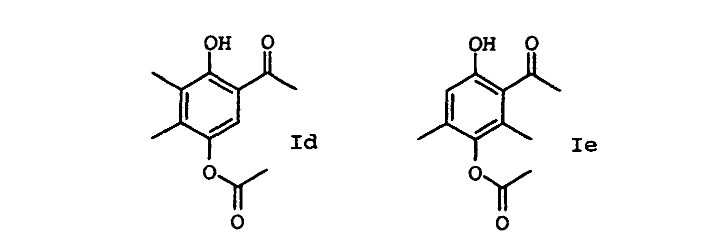

## Claims

1. A process for preparing monoacetylated hydroquinone compounds of the formula I 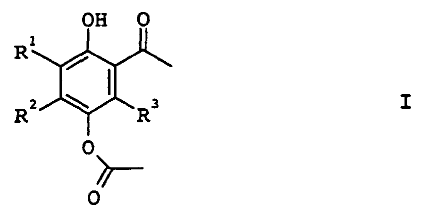 where
R¹, R² or R³
is, independently of one another, hydrogen or methyl,
which comprises reacting diacetylphenol compounds of the formula II 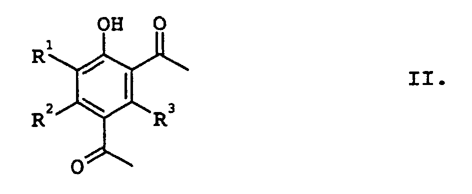 with peroxo compounds.

2. A process as claimed in claim 1, wherein the diacetylphenol compounds of the formula II are reacted with peroxo compounds in the presence of an acid.

3. A process as claimed in claim 2, wherein H₂O₂ or peracids or salts thereof are used as peroxo compounds.

4. A process as claimed in claim 2 or 3, wherein an optionally halogenated carboxylic acid is used as acid.

5. A process as claimed in claim 1, wherein the diacetylphenol compounds of the formula II are prepared by reacting phenol compounds of the formula III 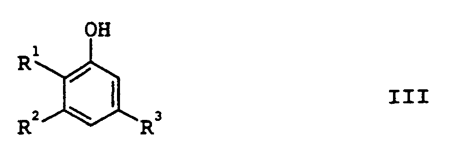 with an acetylating agent in the presence of an acidic catalyst to give the diacetylphenol compounds of the formula II

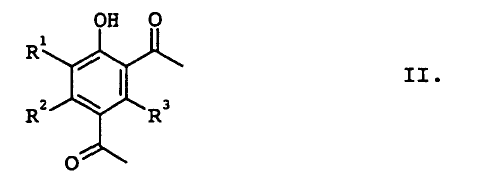

6. A compound of the formulae Ia to Ie

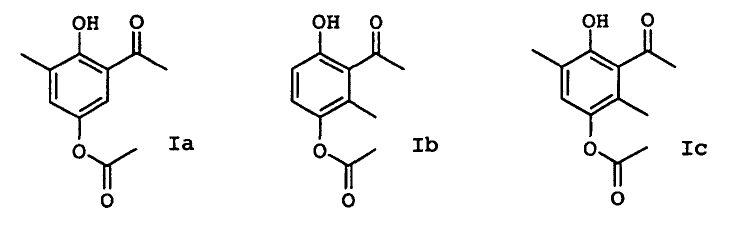

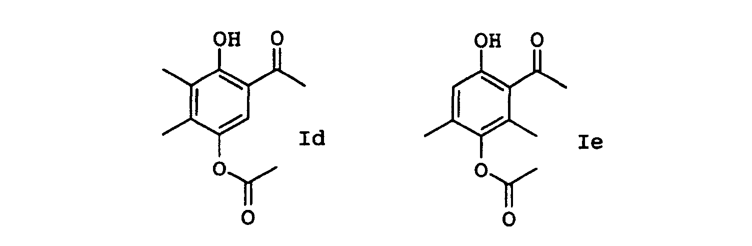

## Revendications

1. Procédé pour la préparation de composés d'hydroquinone monoacétylés de formule I 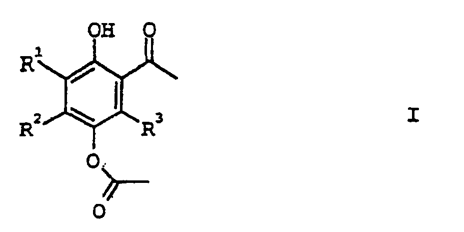 où
R¹, R² ou R³
représentent indépendamment l'un de l'autre l'hydrogène ou un méthyle,
**caractérisé par le fait qu'**on fait réagir des composés de diacétylphénol de formule II 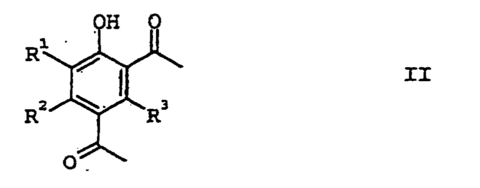 avec des composés peroxo.

2. Procédé selon la revendication 1, **caractérisé par le fait qu'**on fait réagir les composés de diacétylphénol de formule II avec des composés peroxo en présence d'un acide.

3. Procédé selon la revendication 2, **caractérisé par le fait qu'**on utilise comme composés peroxo H₂O₂ ou des peracides ou leurs sels.

4. Procédé selon la revendication 2 ou 3, **caractérisé par le fait qu'**on utilise comme acide un acide carboxylique, éventuellement halogéné.

5. Procédé selon la revendication 1, **caractérisé par le fait qu'**on prépare les composés de diacétylphénol de formule II en faisant réagir des composés phénoliques de formule III 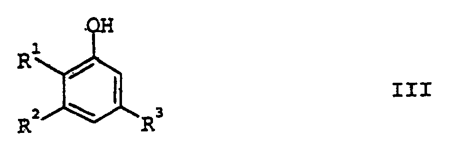 avec un agent d'acétylation en présence d'un catalyseur acide pour former les composés de diacétylphénol de formule II

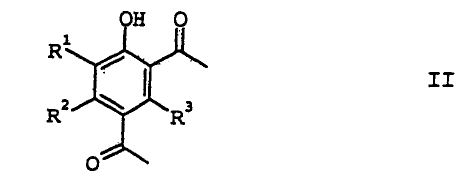

6. Composés de formule Ia à Ie.
